# EUROPÄISCHE PATENTSCHRIFT

(11) **EP 1 843 814 B1**
(45) Veröffentlichungstag und Bekanntmachung des Hinweises auf die Patenterteilung: **21.05.2008**
(21) Anmeldenummer: 06703832.3
(22) Anmeldetag: 21.01.2006
(51) Int. Cl.: A61N 1/36

(54) **VORRICHTUNG ZUR TRANSKUTANEN STIMULATION EINES NERVS DES MENSCHLICHEN KÖRPERS**
DEVICE FOR THE TRANSDERMAL STIMULATION OF A NERVE OF THE HUMAN BODY
DISPOSITIF DE STIMULATION TRANSCUTANEE D'UN NERF DU CORPS HUMAIN

(30) Priorität: 26.01.2005 DE 102005003735
(43) Veröffentlichungstag der Anmeldung: 17.10.2007
(73) Patentinhaber: cerboMed GmbH, 91052 Erlangen (DE)
(72) Erfinder: KRAUS, Thomas, 90408 Nürnberg (DE); DIETRICH, Stefan, 91056 Erlangen (DE)
(74) Vertreter: Gosdin, Michael
(86) Internationale Anmeldenummer: PCT/EP2006/000513
(87) Internationale Veröffentlichungsnummer: WO 2006/079484

(56) Entgegenhaltungen:
- WO-A-20/04000413
- US-A- 4 267 838
- US-A- 4 319 584
- US-A- 4 934 378
- US-A- 5 673 692

## Beschreibung

Die Erfindung betrifft eine Vorrichtung zur transkutanen Stimulation eines Nervs des menschlichen Körpers, die eine Stimulationselektrode und eine Referenzelektrode zur transkutanen Nervenstimulation aufweist, wobei die Stimulationselektrode und die Referenzelektrode mit einer Steuereinheit in Verbindung stehen und von dieser mit einem elektrischen Strom beaufschlagt werden können, wobei die Stimulationselektrode und die Referenzelektrode in oder an einem Gehäuse angeordnet sind, das zur Anbringung am oder im menschlichen Ohr ausgebildet ist.

Vorrichtungen der gattungsgemäßen Art sind in vielfältiger Weise bekannt. Eine transkutane Nerven- und Muskelstimulation wird beispielsweise in der Sportmedizin zur Anregung eines Muskels eingesetzt, wobei hierzu Elektroden auf die Haut aufgesetzt bzw. aufgeklebt werden. Von Vorteil ist, dass es zu keiner Traumatisierung der Hautoberfläche kommt, wenn die Elektrode in Position gebracht wird (nicht-invasive Aufbringung der Elektrode). Dem stehen ebenfalls vorbekannte Möglichkeiten gegenüber, bei denen Elektroden in bzw. unter die Haut eingepflanzt werden.

Bei den Vorrichtungen zur transkutanen Nervenstimulation ist es bekannt, durch invasive oder nicht-invasive elektrische Reizung der Nerven Einfluss auf deren neuroelektrische Qualität und damit auf die Funktion der zu stimulierenden Nerven zu nehmen. Ziel dieser Vorgehensweise ist es, psychovegetative Veränderungen wie beispielsweise Stressabbau hervorzurufen oder neuropsychiatrische Störungen zu behandeln.

Besondere Bedeutung hat seit langem die Stimulation des Nervus vagus. Er ist als zehnter Hirnnerv der Hauptnerv des parasympathischen Systems. Außerdem ist er an der motorischen Steuerung von Kehlkopf und Rachen beteiligt und übermittelt Geschmacksempfindungen vom Zungengrund sowie Berührungsempfindungen aus dem Rachen, dem Kehlkopf und einem Teil des äußeren Gehörgangs (Ramus auricularis).

Insbesondere die invasive Vagus-Nerv-Stimulation gilt mittlerweile als etabliertes Therapie-Verfahren in der Neurologie zur Epilepsie-Behandlung, wozu auf Penry JK, Dean JC: Prevention of intractable partial seizures by intermittent vagal stimulation in humans: preliminary results. Epilepsia 1990; 31 Suppl 2: 40-43, und auf Uthman BM, Wilder BJ, Hammond EJ, Reid SA: Efficacy and safety of vagus nerve stimulation in patients with complex partial seizures. Epilepsia 1990; 31 Suppl 2:44-50, hingewiesen wird.

Hierbei wird dem Patienten neurochirurgisch im linken Halsbereich der Vagusnerv operativ freigelegt und mit einem Stromleiter als Elektrode umwickelt. Das Gerät zur Strom-Impuls-Erzeugung wird im linken Schulterbereich unter der Haut implantiert. Der Vagusnerv-Stimulator kann später mittels eines elektromagnetischen Feldes von außen programmiert werden. Durch die elektrische Vagusnerv-Reizung erfolgt eine Stimulation des Gehirns in verschiedenen Bereichen, was sich in bildgebenden Verfahren nachweisen lässt. Neben der Wirksamkeit bei Epilepsie führt die Stimulationsbehandlung außerdem zu psychischen Wirkungen, z. B. zu antidepressiven Effekten, s. hierzu Elger G, Hoppe C, Falkai P, Rush AJ, Elger CE: Vagus nerve stimulation is associated with mood improvements in epilepsy patients. Epilepsy Res 2000; 42:203-210.

Deshalb wird das Verfahren in jüngster Zeit auch in der Psychiatrie angewandt und bewährte sich bereits in der Behandlung von sonst therapieresistenten schweren Depressionen (s. hierzu Carpenter LL, Friehs GM, Price LH: Cervical vagus nerve stimulation for treatment-resistant depression. Neurosurg Clin N Am 2003; 14:275-282, Goodnick PJ, Rush AJ, George MS, Marangell LB, Sackeim HA: Vagus nerve stimulation in depression. Expert Opin Pharmacother 2001; 2:1061-1063, und Rush AJ, George MS, Sackeim HA, Marangell LB, Husain MM, Giller C, Nahas Z, Haines S, Simpson RK, Jr., Goodman R: Vagus nerve stimulation (VNS) for treatment-resistant depressions: a multicenter study. Biol Psychiatry 2000; 47:276-286).

Zur Stimulation von Nerven im allgemeinen wurden bereits verschiedene Lösungsvorschläge gemacht.

Die US-A-4319584 offenbart eine Vorrichtung gemäss dem Oberbegriff des Patentanspruches 1.

Die US 5,458,625 zeigt eine Vorrichtung der eingangs genannten Art zur Nervenstimulation mittels elektrischer Impulse. Dabei werden die Stromimpulse mittels Elektroden eingebracht, die am Ohrläppchen des Patienten angebracht sind.

Andere Lösungen zur Einbringung elektrischer Impulse in den menschlichen Körper sind aus der JP 101 08 913 A**,** aus der DE 39 18 329 A1 und aus der FR 2 717 699 A1 bekannt.

Die EP 0 645162 B1 beschreibt ein Stimulationsgerät zur Stimulation von Muskeln und Nerven, welches einen Funktionsgenerator zum Erzeugen einer Wellenform mit einer Sequenz von Pulsen umfasst. Es ist besonders für die Schmerztherapie vorgesehen. Die EP 0 757 573 A1 und die EP 1064 047 A1 beschreiben Systeme und Verfahren zur elektrischen Nervenstimulation im allgemeinen.

In der EP 1145 736 A2 wird ein implantierbarer, multimodaler Neurostimulator vorgeschlagen. Die EP 0972 538 A2 beschreibt ein System zur Abgabe einer elektrischen Reizung an einem Teil des Nervensystems mittels einer Nadelelektrode. Die EP 1 048 319 A2 stellt ein System zur selektiven Aktivierung von Gehirnneuronen, Wirbelsäulenparenchyma oder periphären Nerven, welches mit einer einführbaren Kanüle arbeitet, vor.

In der EP 1 022 034 A1 wird ein Verfahren und eine Vorrichtung zur Stimulation von Muskeln oder Nervengewebe durch Erzeugung von Impulssignalen beschrieben. Die EP 1393 773 A1 beschreibt einen externen Nervenstimulator des Nervus phrenicus mittels einer ösophagealen Elektrode. Die EP 0 962 234 A1 beschreibt eine Vorrichtung zur elektrischen Nervstimulation, wobei eine Platzierung der Elektroden nicht offenbart wird.

Die WO 97/45160 beschreibt eine Vorrichtung zur Modulation der neuronalen Gehirnplastizität. In der WO 01/00273 wird eine non-invasive Methode und Vorrichtung zur Herzfrequenzstabilisation mittels Hautelektroden beschrieben. Die EP 1420 854 A2 verwendet eine Zwerchfell-Elektrode zur Behandlung neuropsychiatrischer Störungen. In der EP 1 418 981 A1 erfolgt schließlich eine Stimulation von Nerven in der Nähe des Zwerchfells zur Therapie von Bewegungsstörungen.

Bei den vorbekannten Lösungen haben sich folgende Umstände als nachteilig erwiesen:

Zahlreiche Verfahren erfordern das invasive Einbringen der Stimulationselektrode und sind neben den üblichen Risiken der Anästhesie und eines chirurgischen Eingriffs besonders mit der Gefahr von Nervenverletzungen und einem Infektionsrisiko verbunden.

Sollte die Therapie keinen Erfolg zeigen, so muss das Stimulationsgerät mit den selben Risiken wie oben beschrieben wieder entfernt werden.

Es besteht daher eine Hemmschwelle seitens der Patienten, sich einer solchen Operation zu unterziehen.

Hinzu kommt unter Umständen das Gefühl, einer im Körper implantierten Maschine ausgeliefert zu sein und sich dieser bei Bedarf nicht in einfacher Weise entledigen zu können.

Die beschriebenen Geräte haben zudem oft eine große, unhandliche Bauweise und sind somit nur für den klinischen Gebrauch geeignet nicht aber für die häusliche Langzeitstimulation.

Stimulationsgerät und Elektroden sind häufig über längere Kabel verbunden, was sich nachteilig auf die Handhabung auswirkt.

Eine Stimulation kann aufgrund der Handhabung (z. B. große Klebeelektroden) nur an einem festen Liegeplatz erfolgen, nicht aber unauffällig und nicht störend beim Verrichten täglicher Arbeiten.

Für einen großflächigen therapeutischen Einsatz der Methode der transkutanen Vagusstimulation ist es wünschenswert, die Technik in eine kleine handliche, möglichst kabellose Vorrichtung zu integrieren, die jederzeit, unkompliziert und unauffällig getragen werden kann.

Der Erfindung liegt daher die Aufgabe zugrunde, eine Vorrichtung der eingangs genannten Art so weiterzubilden, dass dieser Wunsch befriedigt wird. D. h. es soll eine Vorrichtung zur Nervenstimulation geschaffen werden, die eine besonders effiziente und einfache Stimulation des Vagusnervs ermöglicht und zwar im Alltag und in einfacher und quasi nicht sichtbarer Weise. Die Vorrichtung soll für den Patienten in besonders einfacher Weise anwendbar sein und es insbesondere ermöglichen, gegebenenfalls schnell vom Körper entfernt werden zu können. Es soll dabei eine effiziente externe, non-invasive Vorrichtung zur Stimulation der Vagusanteile vorgeschlagen werden, die sich durch einen hohen Tragekomfort auszeichnet, wobei die Stimulation jederzeit und unkompliziert erfolgen können soll. Zudem soll dem Patienten die Möglichkeit einer Kontrolle über die Therapie gegeben werden. Weiterhin soll die Vorrichtung auf ein einfaches, stabiles und sicheres Stimulationsverfahren abstellen.

Die Lösung dieser Aufgabe durch die Erfindung ist dadurch gekennzeichnet, dass zum optimalen Positionieren der Elektroden das Gehäuse einen bügelförmigen Fortsatz aufweist, der zur Einführung in den Gehörgang ausgebildet ist, wobei der bügelförmige Fortsatz der Form des Gehörgangeinganges bzw. des äußeren Gehörgangs nachgebildet ist und wobei am Ende des bügelförmigen Fortsatzes ein Elektrodenkopf angeordnet ist, der zwei Kontaktstellen für die beiden Elektroden aufweist.

Dabei ist die Vorrichtung zur Stimulation des Nervus vagus im Bereich des äußeren Gehörgangs und/oder der Ohrmuschel ausgebildet und geeignet.

Bevorzugt ist die Steuereinheit in dem Gehäuse angeordnet. Es kann aber auch vorgesehen sein, dass die Steuereinheit von dem Gehäuse entfernbar ist und mit den Elektroden in Verbindung steht. Dabei kann die Verbindung eine Drahtverbindung sein; möglich ist aber auch eine drahtlose Verbindung, beispielsweise eine Funkverbindung.

Der Elektrodenkopf besteht mit Vorteil aus einem weichen Material, insbesondere aus weichbleibendem Silikon. Die Kontaktstellen können durch Metallkugeln gebildet werden. Sie können auch durch flache Oberflächenelektroden gebildet werden. Weiterhin ist es möglich, dass die Kontaktstellen durch ein Element aus einem Material mit elektrischer Oberflächenleitfähigkeit, insbesondere aus einem Schwamm mit Graphiteinlagerungen, gebildet werden.

Die Steuereinheit kann zur Beeinflussung der Frequenz eines durch die Elektroden fließenden Wechselstroms geeignet sein. Entsprechendes gilt für die Beeinflussung der Höhe des durch die Elektroden fließenden Stroms, für die Beeinflussung der Länge von Impulsen des durch die Elektroden fließenden Stroms, für die Beeinflussung von zeitlichen Stimulationsintervallen des durch die Elektroden fließenden Stroms und/oder für die Beeinflussung des zeitlichen Verlaufs des durch die Elektroden fließenden Stroms.

Vorzugsweise ist in der Vorrichtung eine wiederaufladbare Batterie angeordnet, die die Steuereinheit mit Strom versorgt.

Weiterhin kann vorgesehen sein, dass die Vorrichtung einen Sensor zur Messung eines physiologischen Parameters des Patienten aufweist. Hierbei kann es sich beispielsweise um den Puls des Patienten oder um die Sauerstoffsättigung des Bluts des Patienten handeln. Weiterhin kann ein Speicherchip zur Speicherung der mittels des Sensors gemessenen Daten vorgesehen werden.

Die Elektroden können in das Ohrstück bzw. in den Kopfhörer einer Mobiltelefon-Freisprecheinrichtung integriert und die Steuereinheit in ein Mobiltelefon integriert sein. Dabei kann vorgesehen werden, dass die Verbindung zwischen Elektroden und Steuereinheit über eine Funkverbindung, insbesondere über eine Blue-Tooth-Verbindung oder eine WLAN-Verbiridung, erfolgt.

Möglich ist es auch, dass die Elektroden in den Kopfhörer eines Musikwiedergabegeräts integriert sind und dass die Steuereinheit in das Musikwiedergabegerät integriert ist.

Da der Vagusnerv auch afferente Bahnen in der Haut des äußeren Gehörgangs besitzt, ist eine elektrische Vagusnerv-Stimulation auch durch die Haut des Ohres hindurch und damit nicht-invasiv mittels einer transkutanen Elektrode möglich. Es ist bereits der Nachweis gelungen, dass eine elektrische Reizung des Vagusnervs über Afferenzen im äußeren Gehörgang zu einem ableitbaren Potential auf der Schädeloberfläche führt (sensorisch evoziertes Potential).

Das vorgeschlagene Konzept stimuliert also die im Bereich des äußeren Gehörgangs verlaufenden Nervenäste (Ramus auricularis) des Nervus vagus und nimmt so Einfluss auf dessen Funktion. Dies gelingt durch die Integration der Technik der transkutanen Vagusnervstimulation in eine am oder hinter dem Ohr zu tragende Stimulationsvorrichtung, welche vom äußeren Erscheinungsbild einem Hörgerät ähnelt.

Externe (non-invasive) Stimulationseinheiten für den Vagusnerv im Ohrbereich existierten bislang nicht. Hier schafft die Erfindung Abhilfe. Die vorbekannten non-invasiven Nervenstimulationsverfahren mittels Stromanwendung dienen der peripheren Nerven- und Muskelreizung zur Schmerztherapie (transkutane elektrische Nervenstimulation - TENS), zum Muskeltraining (elektrische Muskelstimulation - EMS) oder zur Elektroakupunktur von bestimmten Meridianpunkten. Keines dieser Verfahren ist dazu vorgesehen, den Vagusnerv im Ohrbereich zu stimulieren, um Veränderungen im Zentralnervensystem hervorzurufen.

Demgegenüber stellt die Erfindung auf die transkutane Vagusnervstimulation im Ohrbereich ab und schlägt hierfür eine besonders einfach anzuwendende Vorrichtung vor.

Mit dem Erfindungsvorschlag wird also eine transkutane Stimulation des Nervus vagus, insbesondere zur Behandlung neuropsychiatrischer Störungen, möglich, wobei eine im oder am äußeren Gehörgang platzierte Stimulationselektrode zur transkutanen Stimulation des Ramus auricularis des Vagusnervs sowie eine im oder am äußeren Gehörgang platzierte Referenzelektrode vorgesehen wird, die vorzugsweise mit einer Steuereinheit verbunden sind, die am oder hinter dem Ohr getragen wird.

Wird das Ohrstück eingesetzt, berühren die Elektroden die Hautoberfläche des äußeren Gehörgangs und können so die sich dort befindlichen Vagusnervanteile stimulieren.

In der Zeichnung ist ein Ausführungsbeispiel der Erfindung dargestellt. Es zeigen:
- Fig. 1: schematisch das Schaltbild einer Vorrichtung zur transkutanen Stimulation des Ramus auricularis des Vagusnervs und
- Fig. 2: das Stimulationsgerät, ausgebildet als HdO-(Hinter-dem-Ohr)-Gerät.

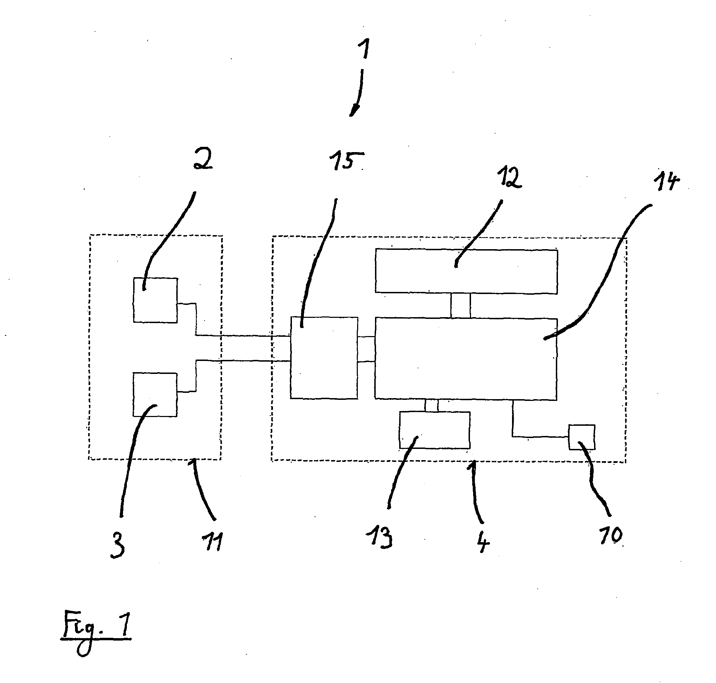

In Fig. 1 ist das Schaltbild einer Vorrichtung 1 zur transkutanen Vagusnervstimulation skizziert. Stimuliert wird insbesondere der Ramus auricularis zur Einflussnahme auf psychovegetative Parameter. Hierdurch kann beispielsweise der Abbau von Stress erfolgen oder es kann positiv auf Depressionen oder andere neuropsychiatrische Störungen Einfluss genommen werden.

Die Vorrichtung 1 besteht grundsätzlich aus der (links in Fig. 1 gestrichelt dargestellten) Stimulationselektrodeneinheit 11 und aus der (rechts in Fig. 1 gestrichelt dargestellten) Steuereinheit 4.

Die Stimulation des Nervs erfolgt über die Stimulationselektrode 2. Als elektrischer Referenzpunkt dient die Referenzelektrode 3. Beide Elektroden 2, 3 bilden die Stimulationselektrodeneinheit 11. Elektroden 2 und 3 zur transkutanen Stimulation sind bekannt, kommerziell erhältlich bzw. leicht herzustellen.

Die Stimulationsfrequenz und die Stimulationsstärke werden von der Steuereinheit 4 vorgegeben und erzeugt. Die Einstellungen dieser Parameter erfolgt über verschiedene Steuerelemente 12. Zur transkutanen Stimulation werden oszillierende Signale benötigt. Diese werden von einem sich in der Steuereinheit 4 befindlichen Oszillator 13 erzeugt. In einer Logik- und Steuerschaltung 14 erfolgt die Verarbeitung der Eingangs- und Ausgangssignale, welche über eine Eingangs- bzw. Ausgangsschaltung 15 der Stimulationselektrodeneinheit 11 zugeführt werden. Die Stromversorgung wird von einer Batterie 10 übernommen.

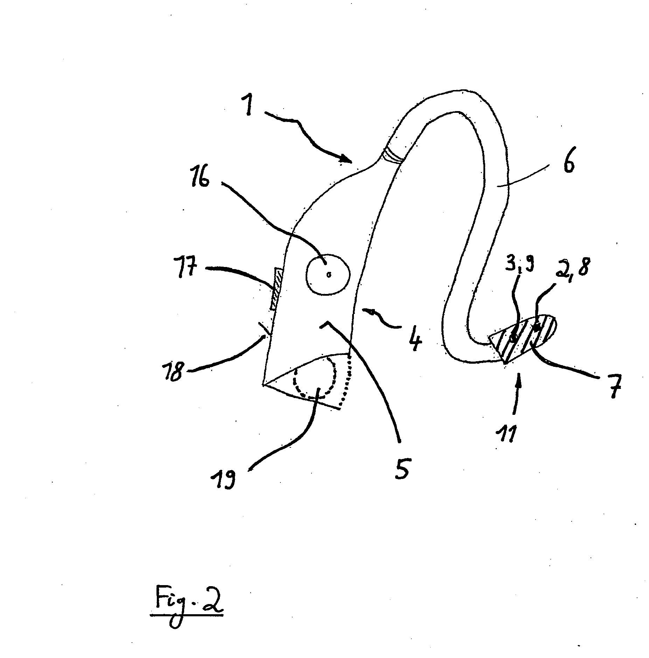

Wie in Fig. 2 zu erkennen ist, ähnelt das Gerät 1 in seiner Bauweise einem hinter dem Ohr getragenen Hörgerät und weist ein Gehäuse 5 auf. Die Stimulationselektrodeneinheit in Form eines Elektrodenkopfs bzw. einer Ohrelektrode 7 wird in den äußeren Gehörgang eingeführt, so dass die Stimulationselektrode 2 und die Referenzelektrode 3 auf der Hautoberfläche zu liegen kommen. Die Verbindung zwischen dem Elektrodenkopf 7 und dem links in Fig. 2 dargestellten Abschnitt des Gehäuses 5 ist als bügelförmiger Fortsatz 6 ausgebildet, welcher auch die gesamten Zu- und Ableitungen zwischen Stimulationselektrodeneinheit und Steuereinheit führt; der bügelförmige Fortsatz 6 wird über den Oberrand der Ohrmuschel gelegt. Am Ende der Verbindung bzw. Überführung befindet sich die Steuereinheit 4 in dem Gehäuse 5 mit einer ungefähren Größe von 5 cm x 2 cm x 1 cm.

In die Steuereinheit 4 eingebaut ist zunächst ein Stimulationsstärkeregler 16 zur Regelung der Amplitude (Stärke) des Stimulationssignals. Große Amplituden stimulieren den Nerv mehr als niedrige Amplituden. Außerdem ist die benötigte Stimulationsstärke interindividuell verschieden.

Ferner ist ein Stimulationsfrequenzregler 17 zur Regelung des Frequenzmusters des Stimulationssignals enthalten. So können schnell aufeinander folgende Signale ebenso angesteuert werden wie Signale, welche mit einem größeren Abstand aufeinander folgen.

Vorhanden ist weiterhin ein Ein/Aus-Schalter 18 zur Aktivierung bzw. Deaktivierung der Vorrichtung 1. Ein Batteriefach 19 dient zur Aufnahme einer kleinen Knopfzell-Batterie vorzugsweise der Größe 13 bis 675.

Ein Beispiel für eine Beaufschlagung des Nervus vagus mit der vorgeschlagenen Vorrichtung sieht wie folgt aus: Der aufgegebene Strom liegt zwischen 0,25 und 1,5 mA. Die Frequenz des Stroms beträgt zwischen 20 und 30 Hz. Die Pulsweite liegt zwischen 250 und 500 µs. Der Strom wird dabei alle 3 bis 5 Minuten für ca. 30 sec. aufgegeben.

Das vorgeschlagene Stimulationsgerät 1 ist sehr klein und eignet sich daher hervorragend für die häusliche Anwendung. Es gibt dem Träger eine große Freiheit, da eine Platzierung hinter dem Ohr sehr vorteilhaft und nicht störend ist.

Die Stimulations- und Referenzelektroden 2, 3 müssen elektrischen Kontakt zu der Hautoberfläche des Patienten haben, wozu Kontaktstellen 8 bzw. 9 dienen, die als metallische Kügelchen ausgebildet sein können. Hiermit liegen die Elektroden 2, 3 an der Innenseite des Tragus, d. h. einem anatomischen Teil der Ohrmuschel, an. Der Abstand zwischen den Kontaktstellen 8, 9 liegt bevorzugt zwischen 1 mm und 15 mm, besonders bevorzugt zwischen 2 mm und 6 mm.

Eine andere Lösungsvariante sieht vor, das Ohrstück weiter in den Gehörgang einzuführen und dort ebenfalls eine Vagusnervstimulation durchzuführen. Hierzu können die Elektroden 2, 3 z. B. als flache Oberflächenelektroden ausgebildet sein. Tiefer im Gehörgang werden weitere Nervenendigungen des Nervus vagus stimuliert.

Die Elektroden 2, 3 sind mit (nicht dargestellten) Kabeln verbunden, welche unsichtbar im Ohrstück geführt werden. Die Kabelverbindungen wiederum sind mit der sich vorzugsweise hinter dem Ohr befindlichen Steuereinheit 4 verbunden. Die Verbindung erfolgt, wie erläutert, über den bügelförmigen Fortsatz 6. Die Einstellung der Stimulationsfrequenz, der Stimulationsstärke, der Impulsdauer sowie der Stimulationsintervalle und der Stromform erfolgt über den Stimulationsfrequenzregler 17.

Ähnlich einem Im-Ohr-Hörgerät kann die gesamte Technik auch in einem Gerät, welches in der Concha des Ohres zu liegen kommt und diese ausfüllt, integriert sein.

Das Gerät wird mit der Batterie 10 mit Strom versorgt und ist somit unabhängig von einer externen Stromquelle. Vorgesehen kann dabei werden, dass die Stromversorgung über eine wiederaufladbare Batterie 10 erfolgt, welche in das Gehäuse 5 integriert ist. Für den Aufladevorgang wird das Gerät 1 in ein spezielles Kästchen gelegt, welches mit einer externen Stromquelle verbunden ist und beispielsweise über Induktion die Batterie 10 über Nacht auflädt.

Das Ohrstück kann zusätzlich mit einem Sensor zur Messung des Pulses und der Sauerstoffsättigung versehen sein, wobei solche Sensoren für die Messung der Atmungsfunktion und des Pulses bekannt und kommerziell erhältlich sind. Die gemessenen Werte können auf einem Speicherchip, welcher sich im Gehäuse 5 hinter dem oder im Ohr befindet, aufgezeichnet werden, um sie später von einem Arzt mittels einer kabellosen Schnittstelle auslesen und per Software auswerten zu können. Über die Änderung der von der Software errechneten Pulsratenvariabilität kann der Arzt wichtige Informationen über die psychovegetative Modulationswirkung der Stimulationsvorrichtung gewinnen und erhält somit auch Kontrolldaten über den Verlauf der Therapie.

Das beschriebene Gerät kann in seiner Bauart nach Standardwerten erfolgen, oder aber das Ohrstück und weitere Teile können individuell gefertigt werden.

Eine alternative Ausführungsform sieht vor, dass der Elektrodenkopf 7 und die Steuereinheit 4 separat gehalten werden und über ein Kabel verbunden sind.

Als eine weitere Alternative kann vorgesehen werden, die Stimulationstechnik in ein Mobiltelefon und dessen Freisprecheinrichtung zu integrieren. Die Steuereinheit 4 und deren Elektronik können hierbei in die Schaltkreise des Mobiltelefons integriert sein. Die Stimulationseinheit 7 mit Stimulations-und Referenzelektrode 2, 3 können in das Ohrstück der Freisprecheinrichtung eingebaut sein. Die Kommunikation zwischen Ohrstück und Mobiltelefon kann sowohl drahtlos, beispielsweise mittels Bluetooth-Technologie, oder über ein Verbindungskabel erfolgen.

Weiterhin ist es möglich, die Technologie in Kopfhörer und Geräte beispielsweise für die digitale Medienwiedergabe zu integrieren. Dies können neben MP3-Playern insbesondere MD-Player oder Discmans sein.

### Bezugszeichenliste:

- 1: Vorrichtung zur transkutanen Stimulation eines Nervs
- 2: Stimulationselektrode
- 3: Referenzelektrode
- 4: Steuereinheit
- 5: Gehäuse
- 6: bügelförmiger Fortsatz
- 7: Elektrodenkopf
- 8: Kontaktstelle
- 9: Kontaktstelle
- 10: Batterie
- 11: Stimulationselektrodeneinheit
- 12: Steuerelemente
- 13: Oszillator
- 14: Logik- und Steuerschaltung
- 15: Eingangs- bzw. Ausgangsschaltung
- 16: Stimulationsstärkeregler
- 17: Stimulationsfrequenzregler
- 18: Ein/Aus-Schalter
- 19: Batteriefach

## Patentansprüche

1. Vorrichtung (1) zur transkutanen Stimulation eines Nervs des menschlichen Körpers, die eine Stimulationselektrode (2) und eine Referenzelektrode (3) zur transkutanen Nervenstimulation aufweist, wobei die eine Stimulationselektrode (2) und die eine Referenzelektrode (3) mit einer Steuereinheit (4) in Verbindung stehen und von dieser mit einem elektrischen Strom beaufschlagt werden können, wobei die eine Stimulationselektrode (2) und die eine Referenzelektrode (3) in oder an einem Gehäuse (5) angeordnet sind, das zur Anbringung am oder im menschlichen Ohr ausgebildet ist,
**dadurch gekennzeichnet,**
**dass** das Gehäuse (5) einen bügelförmigen Fortsatz (6) aufweist, der zur Einführung in den Gehörgang ausgebildet ist, wobei der bügelförmige Fortsatz (6) der Form des Gehörgangeinganges bzw. des äußeren Gehörgangs nachgebildet ist und wobei am Ende des bügelförmigen Fortsatzes (6) ein Elektrodenkopf (7) angeordnet ist, der zwei Kontaktstellen (8, 9) für die beiden Elektroden (2, 3) aufweist.

2. Vorrichtung nach Anspruch 1, **dadurch gekennzeichnet, dass** sie zur Stimulation des Nervus vagus im Bereich des äußeren Gehörgangs und/oder der Ohrmuschel ausgebildet und geeignet ist.

3. Vorrichtung nach Anspruch 1 oder 2, **dadurch gekennzeichnet, dass** die Steuereinheit (4) in dem Gehäuse (5) angeordnet ist.

4. Vorrichtung nach Anspruch 1 oder 2, **dadurch gekennzeichnet, dass** die Steuereinheit (4) von dem Gehäuse (5) entfernbar ist und mit den Elektroden (2, 3) in Verbindung steht.

5. Vorrichtung nach Anspruch 4, **dadurch gekennzeichnet, dass** die Verbindung eine Drahtverbindung ist.

6. Vorrichtung nach Anspruch 4, **dadurch gekennzeichnet, dass** die Verbindung drahtlos ist, insbesondere eine Funkverbindung ist.

7. Vorrichtung nach einem der Ansprüche 1 bis 6, **dadurch gekennzeichnet, dass** der Elektrodenkopf aus einem weichen Material besteht, insbesondere aus weichbleibendem Silikon.

8. Vorrichtung nach einem der Ansprüche 1 bis 7, **dadurch gekennzeichnet, dass** die Kontaktstellen (8, 9) durch Metallkugeln gebildet werden.

9. Vorrichtung nach einem der Ansprüche 1 bis 7, **dadurch gekennzeichnet, dass** die Kontaktstellen (8, 9) durch flache Oberflächenelektroden gebildet werden.

10. Vorrichtung nach einem der Ansprüche 1 bis 7, **dadurch gekennzeichnet, dass** die Kontaktstellen (8, 9) durch ein Element aus einem Material mit elektrischer Oberflächenleitfähigkeit, insbesondere aus einem Schwamm mit Graphiteinlagerungen, gebildet werden.

11. Vorrichtung nach einem der Ansprüche 1 bis 10, **dadurch gekennzeichnet, dass** die Steuereinheit (4) zur Beeinflussung der Frequenz eines durch die Elektroden (2, 3) fließenden Wechselstroms geeignet ist.

12. Vorrichtung nach einem der Ansprüche 1 bis 11, **dadurch gekennzeichnet, dass** die Steuereinheit (4) zur Beeinflussung der Höhe des durch die Elektroden (2, 3) fließenden Stroms geeignet ist.

13. Vorrichtung nach einem der Ansprüche 1 bis 12, **dadurch gekennzeichnet, dass** die Steuereinheit (4) zur Beeinflussung der Länge von Impulsen des durch die Elektroden (2, 3) fließenden Stroms geeignet ist.

14. Vorrichtung nach einem der Ansprüche 1 bis 13, **dadurch gekennzeichnet, dass** die Steuereinheit (4) zur Beeinflussung von zeitlichen Stimulationsintervallen des durch die Elektroden (2, 3) fließenden Stroms geeignet ist.

15. Vorrichtung nach einem der Ansprüche 1 bis 14, **dadurch gekennzeichnet, dass** die Steuereinheit (4) zur Beeinflussung des zeitlichen Verlaufs des durch die Elektroden (2, 3) fließenden Stroms geeignet ist.

16. Vorrichtung nach einem der Ansprüche 1 bis 15, **dadurch gekennzeichnet, dass** in der Vorrichtung eine wiederaufladbare Batterie (10) angeordnet ist, die die Steuereinheit (4) mit Strom versorgt.

17. Vorrichtung nach einem der Ansprüche 1 bis 16, **dadurch gekennzeichnet, dass** sie einen Sensor zur Messung eines physiologischen Parameters des Patienten aufweist.

18. Vorrichtung nach Anspruch 17, **dadurch gekennzeichnet, dass** der physiologische Parameter der Puls des Patienten ist.

19. Vorrichtung nach Anspruch 17, **dadurch gekennzeichnet, dass** der physiologische Parameter die Sauerstoffsättigung des Bluts des Patienten ist.

20. Vorrichtung nach einem der Ansprüche 17 bis 19, **gekennzeichnet durch** einen Speicherchip zur Speicherung der mittels des Sensors gemessenen Daten.

21. Vorrichtung nach einem der Ansprüche 1 bis 20, **dadurch gekennzeichnet, dass** die Elektroden (2, 3) in das Ohrstück bzw. in den Kopfhörer einer Mobiltelefon-Freisprecheinrichtung integriert sind und dass die Steuereinheit (4) in ein Mobiltelefon integriert ist.

22. Vorrichtung nach Anspruch 21, **dadurch gekennzeichnet, dass** die Verbindung zwischen Elektroden (2, 3) und Steuereinheit (4) über eine Funkverbindung, insbesondere über eine Blue-Tooth-Verbindung oder eine WLAN-Verbindung, erfolgt.

23. Vorrichtung nach einem der Ansprüche 1 bis 22, **dadurch gekennzeichnet, dass** die Elektroden (2, 3) in den Kopfhörer eines Musikwiedergabegeräts integriert sind und dass die Steuereinheit (4) in das Musikwiedergabegerät integriert ist.

## Claims

1. Device (1) for transcutaneous stimulation of a nerve of the human body, which comprises one stimulation electrode (2) and one reference electrode (3) for transcutaneous nerve stimulation, wherein the one stimulation electrode (2) and the one reference electrode (3) being connected to a control unit (4) and being able to be supplied with an electrical current from the latter, wherein the one stimulation electrode (2) and the one reference electrode (3) being arranged in or on a housing (5) which is designed to be fitted on or in the human ear,
**characterized in**
**that** the housing (5) has a bow-shaped extension piece (6) designed to be inserted into the auditory canal, said bow-shaped extension piece (6) matching the shape of the entrance to the auditory canal or of the external auditory canal, and wherein an electrode head (7) is arranged at the end of the bow-shaped extension piece (6), which has two contact points (8, 9) for the two electrodes (2, 3).

2. Device according to Claim 1, **characterized in that** it is designed and suitable for stimulation of the vagus nerve in the area of the external auditory canal and/or the auricle.

3. Device according to Claim 1 or 2, **characterized in that** the control unit (4) is arranged in the housing (5).

4. Device according to Claim 1 or 2, **characterized in that** the control unit (4) is removable from the housing (5) and is connected to the electrodes (2, 3).

5. Device according to Claim 4, **characterized in that** the connection is a wired connection.

6. Device according to Claim 4, **characterized in that** the connection is wireless, and in particular is a radio connection.

7. Device according to one of Claims 1 to 6, **characterized in that** the electrode head is made of a soft material, in particular of permanently soft silicone.

8. Device according to one of Claims 1 to 7, **characterized in that** the contact points (8, 9) are formed by metal balls.

9. Device according to one of Claims 1 to 7, **characterized in that** the contact points (8, 9) are formed by flat surface electrodes.

10. Device according to one of Claims 1 to 7, **characterized in that** the contact points (8, 9) are formed by an element made of a material with electrical surface conductivity, in particular of a sponge with graphite inserts.

11. Device according to one of Claims 1 to 10, **characterized in that** the control unit (4) is able to influence the frequency of an alternating current flowing through the electrodes (2, 3).

12. Device according to one of Claims 1 to 11, **characterized in that** the control unit (4) is able to influence the level of the current flowing through the electrodes (2, 3).

13. Device according to one of Claims 1 to 12, **characterized in that** the control unit (4) is able to influence the length of impulses of the current flowing through the electrodes (2, 3).

14. Device according to one of Claims 1 to 13, **characterized in that** the control unit (4) is able to influence the stimulation time intervals of the current flowing through the electrodes (2, 3).

15. Device according to one of Claims 1 to 14, **characterized in that** the control unit (4) is able to influence the time profile of the current flowing through the electrodes (2, 3).

16. Device according to one of Claims 1 to 15, **characterized in that** a rechargeable battery (10) is arranged in the device and supplies current to the control unit (4).

17. Device according to one of Claims 1 to 16, **characterized in that** it comprises a sensor for measuring a physiological parameter of the patient.

18. Device according to Claim 17, **characterized in that** the physiological parameter is the patient's pulse.

19. Device according to Claim 17, **characterized in that** the physiological parameter is the oxygen saturation of the patient's blood.

20. Device according to one of Claims 17 to 19, **characterized by** a memory chip for storing the data measured by means of the sensor.

21. Device according to one of Claims 1 to 20, **characterized in that** the electrodes (2, 3) are integrated into the earpiece, or into the headset of a hands-free mobile telephone unit, and **in that** the control unit (4) is integrated into a mobile telephone.

22. Device according to Claim 21, **characterized in that** the connection between electrodes (2, 3) and control unit (4) is established via a radio connection, in particular via a Bluetooth connection or a WLAN connection.

23. Device according to one of Claims 1 to 22, **characterized in that** the electrodes (2, 3) are integrated into the headphones of a music playback system, and **in that** the control unit (4) is integrated into the music playback system.

## Revendications

1. Dispositif (1) de stimulation transcutanée d'un nerf du corps humain, lequel dispositif présente une électrode de stimulation (2) et une électrode de référence (3) pour la stimulation transcutanée d'un nerf, l'électrode de stimulation (2) et l'électrode de référence (3) étant reliées à une unité de commande (4) et pouvant être alimentées par cette dernière en courant électrique, l'électrode de stimulation (2) et l'électrode de référence (3) étant disposées dans ou sur un boîtier (5) configuré pour être placé sur ou dans l'oreille humaine,
**caractérisé en ce que**
le boîtier (5) présente un prolongement (6) en étrier qui est configuré pour être inséré dans le conduit auditif, le prolongement (6) en étrier imitant la forme de l'entrée du conduit auditif ou du conduit auditif extérieur, et une tête d'électrode (7) qui présente deux emplacements de contact (8, 9) pour les deux électrodes (2, 3) et disposée à l'extrémité du prolongement (6) en forme d'étrier.

2. Dispositif selon la revendication 1, **caractérisé en ce qu'**il est configuré et convient pour stimuler le nerf vague dans la zone du conduit auditif extérieur et/ou du pavillon de l'oreille.

3. Dispositif selon les revendications 1 ou 2,
**caractérisé en ce que** l'unité de commande (4) est disposée dans le boîtier (5).

4. Dispositif selon les revendications 1 ou 2, **caractérisé en ce que** l'unité de commande (4) peut être sortie du boîtier (5) et est reliée aux électrodes (2, 3).

5. Dispositif selon la revendication 4, **caractérisé en ce que** la liaison est une liaison filaire.

6. Dispositif selon la revendication 4, **caractérisé en ce que** la liaison est une liaison sans fil et en particulier une liaison radio.

7. Dispositif selon l'une des revendications 1 à 6, **caractérisé en ce que** la tête d'électrode est constituée d'un matériau déformable et en particulier de silicone malléable.

8. Dispositif selon l'une des revendications 1 à 7, **caractérisé en ce que** les emplacements de contact (8, 9) sont formés par des billes métalliques.

9. Dispositif selon l'une des revendications 1 à 7, **caractérisé en ce que** les emplacements de contact (8, 9) sont formés par des électrodes de surface planes.

10. Dispositif selon l'une des revendications 1 à 7, **caractérisé en ce que** les emplacements de contacts (8, 9) sont formés d'un élément en un matériau qui présente une conductivité électrique de surface et en particulier d'une éponge dans laquelle du graphite est incorporé.

11. Dispositif selon l'une des revendications 1 à 10, **caractérisé en ce que** l'unité de commande (4) convient pour agir sur la fréquence d'un courant alternatif qui circule dans les électrodes (2, 3).

12. Dispositif selon l'une des revendications 1 à 11, **caractérisé en ce que** l'unité de commande (4) convient pour agir sur le niveau du courant qui circule dans les électrodes (2, 3).

13. Dispositif selon l'une des revendications 1 à 12, **caractérisé en ce que** l'unité de commande (4) convient pour agir sur la longueur des impulsions du courant qui circule dans les électrodes (2, 3).

14. Dispositif selon l'une des revendications 1 à 13, **caractérisé en ce que** l'unité de commande (4) convient pour agir sur les intervalles temporels de stimulation du courant qui circule dans les électrodes (2, 3).

15. Dispositif selon l'une des revendications 1 à 14, **caractérisé en ce que** l'unité de commande (4) convient pour agir sur l'évolution dans le temps du courant qui circule dans les électrodes (2, 3).

16. Dispositif selon l'une des revendications 1 à 15, **caractérisé en ce qu'**une batterie rechargeable (10) qui alimente l'unité de commande (4) en courant est disposée dans le dispositif.

17. Dispositif selon l'une des revendications 1 à 16, **caractérisé en ce qu'**il présente une sonde de mesure d'un paramètre physiologique du patient.

18. Dispositif selon la revendication 17, **caractérisé en ce que** le paramètre physiologique est le pouls du patient.

19. Dispositif selon la revendication 17, **caractérisé en ce que** le paramètre physiologique est la saturation en oxygène du sang du patient.

20. Dispositif selon l'une des revendications 17 à 19, **caractérisé par** une puce de mémoire qui conserve les données mesurées au moyen de la sonde.

21. Dispositif selon l'une des revendications 1 à 20, **caractérisé en ce que** les électrodes (2, 3) sont intégrées dans l'oreillette ou dans l'écouteur d'un dispositif de téléphonie mobile dit "mains libres" et en ce l'unité de commande (4) est intégrée dans un téléphone mobile.

22. Dispositif selon la revendication 21, **caractérisé en ce que** la liaison entre les électrodes (2, 3) et l'unité de commande (4) s'effectue par liaison radio, en particulier une liaison dite "Blue Tooth" ou une liaison dite "WLAN".

23. Dispositif selon l'une des revendications 1 à 22, **caractérisé en ce que** les électrodes (2, 3) sont intégrées dans les écouteurs d'un appareil de reproduction de musique et **en ce que** l'unité de commande (4) est intégrée dans l'appareil de reproduction de musique.
